# FASCICULE DE BREVET EUROPEEN

(11) **EP 1 965 808 B1**
(45) Date de publication et mention de la délivrance du brevet: **15.08.2012**
(21) Numéro de dépôt: 06847181.2
(22) Date de dépôt: 19.12.2006
(51) Int. Cl.: A61K 31/704, A61K 31/728, A61K 31/702, A61K 8/73, A61P 17/00, A61K 31/07, A61K 9/08, A61K 9/06

(54) **PREPARATIONS PHARMACEUTIQUES OU COSMETIQUES POUR APPLICATION TOPIQUE ET/OU PARENTERALE, LEURS PROCEDES DE PREPARATION, ET LEURS UTILISATIONS**
PHARMAZEUTISCHE ODER KOSMETISCHE ZUBEREITUNGEN FÜR TOPISCHE UND/ODER PARENTERALE ANWENDUNG, ZUBEREITUNGSVERFAHREN UND VERWENDUNG
PHARMACEUTICAL OR COSMETIC PREPARATIONS FOR TOPICAL AND/OR PARENTERAL APPLICATION, PROCESSES FOR THE PREPARATION THEREOF, AND USES THEREOF

(30) Priorité: 21.12.2005 FR 0513055
(43) Date de publication de la demande: 10.09.2008
(73) Titulaire: GALDERMA RESEARCH & DEVELOPMENT, 06410 Biot (FR)
(72) Inventeur: MOUTET, Marc, F-94230 Cachan (FR); YADAN, Jean-Claude, F-93100 Montreuil Sous Bois (FR)
(74) Mandataire: Boulard, Denis
(86) Numéro de dépôt international: PCT/FR2006/051391
(87) Numéro de publication internationale: WO 2007/074288

(56) Documents cités:
- EP-A- 0 608 433
- WO-A-2005/039532
- US-A- 6 024 941

## Description

La présente invention se rapporte à des préparations pour application topique et/ou parentérale comprenant, dans un milieu physiologiquement acceptable, de l'acide hyaluronique, des procédés de fabrication de telles préparations, et leurs utilisations en tant que médicament, lesdites préparations étant destinées au traitement des affections dermatologiques, en particulier, au traitement par comblement des rides, ridules, déplétions fibroblastiques et toutes cicatrices.

Le vieillissement de la peau est une des modifications les plus visibles du processus de sénescence. De plus, la peau se retrouve exposée à de nombreux facteurs susceptibles d'accélérer ce processus physiologique. On distingue deux types différents de vieillissement cutané. D'une part, le vieillissement intrinsèque, qu'il est plus facile d'évaluer sur des zones qui normalement ne sont pas exposées au soleil et, d'autre part, le vieillissement extrinsèque, provoqué par l'interaction de facteurs environnementaux, notamment les rayons UV. Ces facteurs environnementaux ont un effet beaucoup plus marqué sur les parties du corps exposées au soleil, surtout chez les personnes de phototype clair. On parle alors également de vieillissement actinique. D'autres facteurs tels que les habitudes alimentaires, le tabagisme, la consommation excessive d'alcool, les maladies chroniques et les dysfonctionnements des glandes endocrines contribuent également à ce vieillissement.

Lors du vieillissement intrinsèque de la peau, la couche cornée est peu modifiée. L'épiderme est atrophique et la jonction dermo-épidermique est aplatie, de sorte que l'adhésion au derme est moins bonne, facilitant la formation de bulles. L'épaisseur du derme est nettement réduite ; il y a moins de vaisseaux sanguins. On observe aussi moins de fibroblastes et leurs capacités de biosynthèse et de prolifération sont diminuées. Les fibres élastiques subissent d'abord des modifications, pour disparaître par la suite.

En ce qui concerne le vieillissement extrinsèque, on observe un épiderme irrégulier, parfois atrophique, parfois hyperplasique, avec des signes de désorganisation et de dysplasie. Les mélanocytes sont plus nombreux à certains endroits, et en nombre réduit à d'autres. Il y a aussi une irrégularité de la distribution de la mélanine dans l'épiderme, suite à des problèmes de transfert des mélanosomes. Le nombre de cellules de Langerhans diminue. Les petits vaisseaux sanguins sont d'abord dilatés, pour ensuite s'amincir et s'atrophier.

Les rides sont les signes les plus visibles du vieillissement. On en distingue de plusieurs natures, notamment les rides superficielles et profondes. Les rides profondes seraient dues aux modifications dermohypodermiques, tandis que les rides superficielles pourraient s'expliquer par des modifications dermiques et éventuellement épidermiques. Les rides sont surtout dues à la perte d'élasticité de la peau. L'atteinte du réseau élastique sous-épidermique donne lieu à une laxité superficielle de la peau vieillie et à un plissement de sa surface. La destruction des fibres élastiques dans le derme réticulaire est responsable de la perte d'élasticité et de la capacité de la peau à reprendre sa forme après étirement. Selon le type, l'intensité et la topographie, un traitement adapté sera possible.

Le traitement des modifications cutanées inesthétiques liées au vieillissement a fait d'énormes progrès ces dernières années.

Un nombre relativement important de substances naturelles ou synthétiques ont d'ores et déjà été décrites en tant qu'implants dermiques, c'est-à-dire en tant que substances injectées directement dans la peau, afin de remédier aux altérations cutanées résultant du vieillissement, de traumatismes ou de maladies.

D'autres alternatives thérapeutiques pour ces applications sont notamment l'injection locale de la toxine botulique (Botox®) ou l'utilisation de techniques laser. Ces différents types de traitement ne sont pas exclusifs et leur combinaison a même été recommandée. Parmi les substances naturelles d'origine humaine, le collagène et l'acide hyaluronique sont celles qui sont à l'origine de la majorité des produits disponibles sur le marché.

L'acide hyaluronique est un polysaccharide naturel ubiquitaire qui existe sous la même forme de la plus simple bactérie à l'Homme. C'est un polymère de disaccharides eux-mêmes composés d'acide D-glucuronique et de N-acétylglucosamine, liés entre eux par des liaisons glycosidiques alternées beta-1,4 et beta-1,3. Les polymères de cette unité récurrente peuvent avoir une taille entre 102 et 104 kDa *in vivo.* L'acide hyaluronique représente notamment un constituant naturel du derme où il joue un rôle important dans l'hydratation et l'élasticité de la peau. Il diminue cependant en quantité et en qualité avec l'âge, entraînant un dessèchement de la peau qui se ride. Il est très hydrosoluble et forme des solutions à haute viscosité dans l'eau. Du fait de ces propriétés particulières, l'acide hyaluronique fait partie des produits pharmaceutiques les plus utilisés.

Toutefois, chez l'Homme, l'acide hyaluronique est très rapidement éliminé du plasma par dégradation. Sa demi-vie plasmatique après injection intraveineuse est très.courte, entre 2,5 et 5,5 minutes, alors que dans la peau, sa demi-vie est de 0,5 à 2 jours selon sa concentration. Son excrétion urinaire est faible, inférieure à 1% de la clairance totale. Chez le lapin, la vitesse d'élimination, dans la peau, a été mesurée (Reed RK, Laurent UB, Fraser JR, Laurent TC. Removal rate of [3H]hyaluronan injected subcutaneously in rabbits. Am J Physiol. 1990 Aug;259(2 Pt 2) :H532-5). Elle est non exponentielle avec une demi-vie de 0,5 à 1 jour quand sa concentration est de 5 mg/ml.

La tolérance de l'acide hyaluronique est très bonne et aucune immunogénicité n'a été associée à cette substance. On retrouve ainsi une incidence d'effets secondaires très faible.

L'utilisation de l'acide hyaluronique, seul ou en association, a ainsi été décrite pour plusieurs applications médicales, telles que par exemple le traitement de l'ostéoarthrite ainsi que l'arthrite rhumatoïde. Des compositions injectables telles que par exemple l'acide hyaluronique seul, le collagène seul ou l'association « acide hyaluronique et collagène » ont également déjà été utilisées en chirurgie réparatrice, dans le cadre de traitement par comblement des rides, ridules, déplétions fibroblastiques et toutes cicatrices.

Actuellement, de nombreux implants dermiques sont utilisés mais aucun n'a encore été considéré comme idéal dans le cadre d'une augmentation tissulaire sûre et saine (Naoum C, Dasiou-Plakida D. Dermal filler materials and botulin toxin Int J Dermatol. 2001 Oct;40(10):609-21).

Cependant, l'acide hyaluronique, en raison de sa biodisponibilité trop faible après injection et sa fréquence d'injection trop élevée, ne peut être utilisé en tant que tel.

Des microémulsions comprenant de l'acide hyaluronique, éventuellement sous forme de sel, un rétinoïde, et des antioxydants ou conservateurs ont été décrites dans la demande WO 2005/039532. Des compositions utiles dans le domaine de la dermatologie comprenant de l'acide hyaluronique, du rétinol, et de l'EDTA ont été décrites dans les demandes EP0608433 et US 6,024,941.

Bien sûr, on a cherché à développer des compositions à base d'acide hyaluronique possédant une très bonne biodisponibilité et susceptibles de mieux résister à l'action des enzymes de dégradation. Ceci permet, notamment, d'espacer les interventions et d'en réduire le nombre.

Ces compositions utilisées en tant qu'implant dermique sont toutes composées d'acide hyaluronique stabilisé et un grand nombre d'entre elles comprennent de l'acide hyaluronique modifié chimiquement dans ce but. En outre, l'acide hyaluronique inclus dans ces produits est majoritairement d'origine non humaine telle que par exemple d'origine aviaire ou bactérienne.

On retrouve ainsi dans ces compositions de nombreux dérivés d'acide hyaluronique modifiés chimiquement sous forme, notamment d'esters, d'amides, ainsi que des dérivés possédant des « ponts intra et/ou interchaînes » (cross-linked).

Cependant, ces modifications affectent les caractéristiques physico-chimiques et les propriétés biologiques de l'acide hyaluronique, ainsi que son devenir après administration. Ces modifications structurelles de l'acide hyaluronique peuvent entraîner des réactions inflammatoires comme le reporte Sopaar CNS, Patrinely JR Ophtalmic plastic and reconstructive surgery 2005 Mar ; 21(2) :151-53.

De plus, bien que supérieure à celle de l'acide hyaluronique naturel, la biodisponibilité de ces dérivés d'acide hyaluronique reste toujours trop courte.

Compte tenu de ce qui précède, un problème que se propose de résoudre l'invention est de réaliser des préparations comprenant de l'acide hyaluronique ayant une meilleure biodisponibilité tout en conservant ses caractéristiques physico-chimiques et ses propriétés biologiques, ainsi qu'un procédé de fabrication d'une telle préparation.

La solution de l'invention à ce problème posé a pour premier objet une composition ou une préparation pharmaceutique ou cosmétique, notamment pour application topique et/ou parentérale comprenant, dans un milieu physiologiquement acceptable, de l'acide hyaluronique, caractérisée en ce qu'elle comprend en outre au moins :
- un rétinoïde et/ou ses sels et/ou ses esters, tels que le rétinyl palmitate, le rétinyl acétate, le rétinyl stéarate, le rétinyl oléate, le rétinyl propionate ou le rétinyl linoléate,
- un pentamère de l'acide hyaluronique, et
- un inhibiteur de la dégradation de l'acide hyaluronique qui est choisi parmi la glycyrrhizine ou l'acide glycyrrhétinique, leurs sels, énantiomères et/ou racémates, pris seuls ou en mélange.

Elle a pour deuxième objet un procédé de fabrication d'une composition ou d'une préparation pharmaceutique ou cosmétique pour application topique et/ou parentérale comprenant, dans un milieu physiologiquement acceptable, de l'acide hyaluronique, et au moins un rétinoïde et/ou ses sels et/ou ses dérivés, un oligosaccharide, et un inhibiteur de la dégradation de l'acide hyaluronique, tels que définis plus haut, caractérisé en ce qu'il comprend l'étape de mélange d'une quantité efficace de l'acide hyaluronique avec au moins un rétinoïde et/ou ses sels et/ou ses dérivés, au moins un oligosaccharide, et au moins un inhibiteur de la dégradation de l'acide hyaluronique, tels que définis plus haut. De préférence, le procédé selon l'invention comprend également une étape de préparation d'un milieu physiologiquement acceptable, dans lequel les actifs sont mélangés.

Enfin, elle a pour troisième objet l'utilisation d'une préparation selon l'invention pour la fabrication d'un médicament destiné au traitement et/ou à la prévention des affections dermatologiques.

Lorsqu'une composition ou préparation pharmaceutique ou cosmétique pour application topique et/ou parentérale comprend, dans un milieu physiologiquement acceptable, de l'acide hyaluronique, et au moins un rétinoïde et/ou ses sels et/ou ses dérivés, un oligosaccharide, et un inhibiteur de la dégradation de l'acide hyaluronique, tels que définis plus haut, elle augmente nettement la biodisponibilité de l'acide hyaluronique, elle permet d'espacer les applications et d'en réduire le nombre et elle présente une efficacité importante dans le comblement des rides, ridules, déplétions fibroblastiques et toutes cicatrices.

L'invention sera mieux comprise à la lecture de la description non limitative qui va suivre, notamment au regard du dessin annexé, dans lequel :
- La figure 1 présente sous une représentation semilogarithmique les résultats d'une étude ayant pour but d'évaluer l'effet inhibiteur de la glycyrrhizine sur l'activité hyaluronidase d'origine bovine.
- La figure 2 présente les résultats d'une étude ayant pour but d'évaluer l'effet de l'association « GLZ + pentamère (NAG-Glucuronic acid)5 + rétinol » sur la néosynthèse d'acide hyaluronique par des kératinocytes humains normaux.

La préparation selon l'invention comprend, dans un milieu physiologiquement acceptable, de l'acide hyaluronique, et au moins un rétinoïde et/ou ses sels et/ou ses dérivés, un oligosaccharide, et un inhibiteur de la dégradation de l'acide hyaluronique, tels que définis plus haut.

La présente invention a également pour objet un produit comprenant, de préférence contenant :
- de l'acide hyaluronique,
- au moins un rétinoïde et/ou ses sels et/ou ses dérivés,
- au moins un oligosaccharide, de préférence un, et
- au moins un inhibiteur de la dégradation de l'acide hyaluronique, de préférence un,
tels que définis plus haut,
comme produit de combinaison pour une utilisation simultanée, séparée ou étalée dans le temps dans le traitement d'affections dermatologiques.
Un tel produit de combinaison est notamment efficace dans le traitement des rides, des ridules, des déplétions fibroblastiques et des cicatrices.

Par produit de combinaison, on entend une composition unique comprenant chacun des composés actifs, mais aussi une composition complexe comprenant au moins deux compositions différentes comprenant chacune une partie des actifs de ladite préparation.

Par actifs, on entend selon la présente invention les composés choisis parmi l'acide hyaluronique, au moins un oligosaccharide, au moins un inhibiteur de la dégradation de l'acide hyaluronique et au moins un rétinoïde et/ou ses sels et/ou ses dérivés, tels que définis plus haut. Ainsi, le produit de combinaison selon l'invention comprend au moins 4 actifs.

A titre d'exemple non limitatif de produit de combinaison selon l'invention, on peut citer :
- une composition unique comprenant de l'acide hyaluronique, un rétinoïde et/ou ses sels et/ou ses dérivés, un oligosaccharide et un inhibiteur de la dégradation de l'acide hyaluronique, tels que définis plus haut-;
- une composition comprenant un seul de ces quatre actifs associée à une composition comprenant au moins les trois autres actifs ;
- une composition comprenant deux de ces quatre actifs, associée à une composition comprenant au moins les deux autres actifs ; et
- une composition comprenant trois de ces quatre actifs associée à une composition comprenant au moins le quatrième actif.

De préférence, le produit de combinaison selon l'invention comprend une composition A comprenant l'acide hyaluronique sous forme de solution injectable (de préférence aqueuse), associée à une composition B comprenant au moins :
- un inhibiteur de la dégradation de l'acide hyaluronique, de préférence la glycyrrhizine,
- un oligosaccharide, de préférence le pentamère (NAC-Glucuronic acid)5, et
- un rétinoïde et/ou ses sels et/ou ses dérivés, de préférence le rétinol,
sous forme de composition pour application topique.

Par milieu physiologiquement acceptable selon l'invention, on entend un milieu compatible avec la peau et éventuellement avec ses phanères (cils, ongles, cheveux) et/ou les muqueuses.

Dans les préparations selon l'invention, l'acide hyaluronique, le rétinoïde et/ou ses sels et/ou ses dérivés, l'oligosaccharide et l'inhibiteur de la dégradation de l'acide hyaluronique, tels que définis plus haut, sont présents en proportions pouvant aller de 0,0000001% à 10%, préférentiellement de 0,00001% à 1% en poids, par rapport au poids total de la préparation. Dans la présente description, et à moins qu'il ne soit spécifié autrement, il est entendu que, lorsque des intervalles de concentrations sont donnés, ils incluent les bornes supérieures et inférieures dudit intervalle.

Les préparations selon l'invention comprennent de l'acide hyaluronique.

Par acide hyaluronique on entend le composé constitué par l'enchaînement d'acide glucuronique et de N-acétyl-glucosamine.

De façon avantageuse, l'acide hyaluronique est naturel.

Par acide hyaluronique naturel, on entend un acide hyaluronique non stabilisé, non modifié chimiquement sous forme, notamment d'esters, d'amides, ou sous forme de dérivés possédant des « ponts intra et/ou interchaines » (cross linked), de telles modifications affectant les caractéristiques physico-chimiques et les propriétés biologiques dudit acide hyaluronique, ainsi que son devenir après administration.

Les préparations selon l'invention comprennent en outre un rétinoïde et/ou ses sels et/ou ses esters, pris seuls ou en mélange.

Parmi les rétinoïdes susceptibles d'entrer dans les préparations selon l'invention, on choisira préférentiellement le rétinol, le rétinal et/ou l'acide rétinoïque, leurs sels et dérivés, pris seuls ou en mélange, plus préférentiellement le rétinol.
Par sel de rétinoïde, on entend notamment un sel de métal alcalin, ou un sel alcalino-terreux, ou un sel d'amine organique. Par dérivé de rétinoïde, on entend les esters,̅ tels que le rétinyl palmitate, le rétinyl acétate, le rétinyl stéarate, le rétinyl oléate, le rétinyl propionate ou encore le rétinyl linoléate.

De façon avantageuse, les rétinoïdes utilisés dans les préparations selon l'invention sont des rétinoïdes existant naturellement dans l'organisme humain.

Les préparations selon l'invention comprennent en outre un oligosaccharide.

Par oligosaccharide, on entend notamment tout oligosaccharide limitant la pénétration de l'acide hyaluronique dans les cellules de la peau, notamment les kératinocytes et les fibroblastes.

Parmi les oligosaccharides, pris seuls ou en mélange, susceptibles d'entrer dans les préparations selon l'invention, on choisira le pentamère de l'acide hyaluronique.

De façon avantageuse, les oligosaccharides utilisés dans les préparations selon l'invention sont des composés existant de façon naturelle dans l'organisme humain.

Dans les préparations selon l'invention, l'oligosaccharide est utilisé à des concentrations comprises entre 10-⁹ M et 10-³ M, préférentiellement entre 10-⁸ M et 10-⁵ M.

Les préparations selon l'invention comprennent en outre un inhibiteur de la dégradation de l'acide hyaluronique.

Par inhibiteur de la dégradation de l'acide hyaluronique, on entend un composé capable de diminuer, voire de bloquer, le catabolisme soit extracellulaire soit intracellulaire de l'acide hyaluronique, préférentiellement un composé capable de diminuer, voire de bloquer, le catabolisme extracellulaire de l'acide hyaluronique, plus préférentiellement un composé capable d'inhiber la hyaluronidase extracellulaire présente dans la peau.

Parmi les inhibiteurs de la dégradation de l'acide hyaluronique, pris seuls ou en mélange, susceptibles d'entrer dans les préparations selon l'invention, on choisira notamment la glycyrrhizine ou l'acide glycyrrhétinique, leurs dérivés

De façon avantageuse, les inhibiteurs de la dégradation de l'acide hyaluronique utilisés dans les préparations selon l'invention sont naturels.

Dans les préparations selon l'invention, l'inhibiteur est utilisé à des concentrations comprises entre 10-⁹ M et 10-² M, préférentiellement entre 10-⁶ M et 10-³ M.

Par dérivés de la glycyrrhizine ou de l'acide glycyrrhétinique, on entend notamment les sels les énantiomères et les racémates desdits composés.

Comme sels desdits composés, on peut citer les sels obtenus par addition desdits composés avec une base inorganique, choisie notamment parmi les hydroxydes de sodium, de lithium, de calcium, de potassium, de magnésium, d'ammonium ou de zinc, les carbonates de métaux alcalins ou alcalino-terreux tels que les carbonates et bicarbonates de sodium, de lithium, de calcium, de potassium, de magnésium, d'ammonium ou de zinc, ou avec une base organique, choisie notamment parmi la méthylamine, la propylamine, la triméthylamine, la diéthylamine, la triéthylamine, la N,N-diméthyléthanolamine, le tris(hydroxyméthyl)-aminométhane, l'éthanolamine, la pyridine, la picoline, la dicyclohexylamine, la morpholine, la proceïne, la lysine, l'arginine, l'histidine, la N-méthylglucamine ou encore les sels de phosphonium tels que les sels d'alkylphosphonium, les sels d'aryl-phosphonium, les sels d'alkyl-arylphosphonium, les alkènyl-arylphosphonium ou les sels d'ammonium quaternaires tels que les sels de tétra-n-butyl-ammonium. De tels sels sont notamment le sel de potassium de l'acide glycyrrhétinique, le sel de sodium de l'acide glycyrrhétinique, ou encore le sel monoammonium de l'acide glycyrrhétinique (ammonium glycyrrhétinate).

De façon avantageuse, les dérivés doivent être d'origine naturelle.

Les composés et leurs dérivés d'origine naturelle sont des composés à l'état pur ou en solution à différentes concentrations, obtenus par différents procédés d'extraction ou d'hydrolyse de matériel biologique d'origine naturelle.

De façon connue, les préparations selon l'invention peuvent contenir également les adjuvants habituels bien connus de l'homme de l'art.

Les préparations selon l'invention sont formulées pour une application par voie topique et/ou parentérale.

Par voie topique, les préparations peuvent se présenter sous toutes les formes galéniques normalement utilisées pour une administration par voie topique. A titre d'exemple non limitatif de préparations topiques, on peut citer des préparations sous forme liquide, pâteuse, ou solide et, plus particulièrement, sous forme d'onguents, de solutions aqueuses, hydroalcooliques ou huileuses, de dispersions du type lotion éventuellement biphasée, de sérum, de gels aqueux, anhydres ou lipophiles, de poudres, de tampons imbibés, de syndets, de lingettes, de sprays, de mousses, de sticks, de shampoings, de compresses, de bases lavantes, d'émulsions de consistance liquide ou semi-liquide du type lait, obtenues par dispersion d'une phase grasse dans une phase aqueuse (H/E) ou inversement (E/H), d'une microémulsion, de suspensions ou émulsions de consistance molle, semi-liquide ou solide du type crème blanche ou colorée, gel ou pommade, de suspensions de microsphères ou nanosphères ou de vésicules lipidiques ou polymériques, ou de microcapsules, micro- ou nanoparticules ou de patchs polymériques ou gélifiés permettant une libération contrôlée.

Par voie parentérale, les préparations selon l'invention peuvent être appliquées par voie sous-cutanée ou intradermique. A titre d'exemple non limitatif de préparations parentérales, on peut citer des préparations sous forme de solutions ou suspensions pour perfusion ou pour injection.

Selon l'invention, les composés constituant la préparation peuvent être administrés selon un même mode d'administration ou selon un mode d'administration combiné.

Par mode d'administration combiné, on entend l'administration de l'un ou de plusieurs composés de la préparation selon l'invention par voie topique combinée à une administration par voie parentérale, notamment par injection sous cutanée ou intradermique du ou des autres composés de la préparation.

De façon avantageuse, une partie des composés est d'abord appliquée par voie topique et une autre partie desdits composés est ensuite appliquée par voie parentérale, ou inversement.

Selon une alternative de l'invention, on peut également appliquer simultanément une partie des composés par voie topique et une autre partie desdits composés par voie parentérale.

A titre d'exemple non limitatif donné simplement à titre d'illustration et qui ne saurait en aucune façon limiter la portée de l'invention, l'acide hyaluronique peut être administré sous forme d'une solution aqueuse injectable, le rétinol, le pentamère d'acide hyalobiuronique et la glycyrrhizine étant administrés sous forme d'une crème.

Dans le cadre d'une administration combinée, les fréquences d'administration peuvent être identiques ou différentes.

A titre d'exemple non limitatif, la fréquence d'administration de l'acide hyaluronique injecté sous forme d'une solution aqueuse injectable peut varier de 1 à 12 mois, préférentiellement de 6 à 12 mois, alors que celles des autres composés de la préparation selon l'invention administrés sous forme de crème peut varier de 1 à 7 jours, préférentiellement de 1 à 3 jours.

Le procédé de fabrication d'une préparation selon l'invention est caractérisé en ce qu'il comprend une étape de mélange d'une quantité efficace d'acide hyaluronique, d'au moins un rétinoïde et/ou ses sels et/ou ses dérivés, d'au moins un oligosaccharide, et d'au moins un inhibiteur de la dégradation de l'acide hyaluronique, tels que définis plus haut. De préférence, ledit procédé comprend une étape de préparation d'un milieu physiologiquement acceptable, dans lequel les actifs sont ajoutés.

Selon un mode particulier de l'invention, le procédés de fabrication d'une préparation comprend les étapes de préparation d'un milieu physiologiquement acceptable et de mélange d'une quantité efficace d'acide hyaluronique, de rétinol, de pentamère de l'acide hyaluronique, et de glycyrrhizine et/ou ses dérivés et/ou analogues.

De façon avantageuse, le procédé de fabrication d'un produit de combinaison selon l'invention comprend une première étape de préparation d'une solution injectable, comprenant le mélange de l'acide hyaluronique avec un milieu physiologiquement acceptable, et une seconde étape de préparation d'une formulation adaptée à la voie topique, comprenant le mélange d'au moins un rétinoïde et/ou ses sels et/ou ses dérivés, avec au moins un oligosaccharide et au moins un inhibiteur de la dégradation de l'acide hyaluronique, tels que définis plus haut, dans un milieu physiologiquement acceptable.

L'invention se rapporte également à l'utilisation d'une préparation telle que décrite précédemment pour la fabrication d'un médicament destiné au traitement et/ou à la prévention des affections dermatologiques.

Plus particulièrement, l'invention se rapporte à l'utilisation d'une préparation telle que décrite précédemment pour la fabrication d'un médicament destiné au traitement des rides, des ridules, des déplétions fibroblastiques et des cicatrices. Un tel médicament est adapté au traitement de la peau ridée et/ou âgée, et vise notamment à en prévenir et/ou à en réduire les effets. Le traitement des rides, ridules, déplétions fibroblastiques et toutes cicatrices se fait notamment par comblement.

En particulier, la préparation selon l'invention peut être appliquée sur les zones du visage ou du front marquées par des rides d'expression.

L'invention se rapporte également à l'utilisation d'une préparation telle que décrite précédemment pour la fabrication d'un médicament destiné à être utilisé en chirurgie réparatrice.

En outre, l'invention se rapporte à l'utilisation d'une préparation selon l'invention au sein d'un implant dermique.

La présente invention va maintenant être illustrée au moyen des exemples suivants.

### Exemple 1 : Effet inhibiteur de la glycyrrhizine (GLZ) sur l'activité hyaluronidase d'origine bovine

### Détermination de la CI50 de GLZ, avec ou sans pré-incubation à 37°C :

La GLZ, à différentes concentrations, est pré-incubée ou non 20 minutes à 37°C en présence de l'enzyme. La réaction enzymatique est déclenchée par ajout de la solution d'acide hyaluronique (temps TO). Après 20 minutes d'incubation, l'acide hyaluronique non hydrolysé est précipité par ajout de la solution acide d'albumine bovine.

Afin de vérifier que l'étape de pré-incubation n'influe pas sur la stabilité de la hyaluronidase, une aliquote d'une solution de l'enzyme est placée à 37°C pendant 20 minutes. Une autre aliquote est conservée dans un bain de glace pendant 19 minutes, puis est incubée à 37°C pendant 1 minute. Une solution d'acide hyaluronique est alors ajoutée dans chaque aliquote (TO). Après 15, 30 ou 45 minutes d'incubation, l'acide hyaluronique non hydrolysé est précipité par ajout de la solution acide d'albumine bovine.

### Mesure de l'activité hyaluronidase d'origine bovine :

Après l'étape de précipitation, la turbidimétrie des solutions est déterminée au spectrophotomètre à une longueur d'onde de 600 nm. La densité optique (DO) de ces solutions est retranchée de la DO d'une solution témoin d'acide hyaluronique (de même concentration) non hydrolysé par l'enzyme. Cette différence de DO, qui est inversement proportionnelle à la concentration d'acide hyaluronique, est utilisée pour mesurer l'activité de la hyaluronidase.

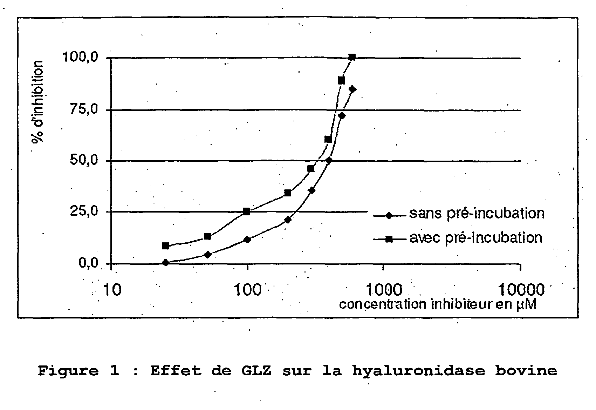

L'effet inhibiteur de la GLZ sur la hyaluronidase bovine est illustré sur la figure 1 (représentation semilogarithmique).

Les résultats obtenus montrent que cet effet est dose-dépendant et que la concentration en GLZ inhibant à 50% (CI50) l'activité hyaluronidase est de 400 µM sans pré-incubation avec l'enzyme.

Lorsque la GLZ est pré-incubée 20 minutes à 37°C en présence de l'enzyme, la CI50 est de 350 µM.

### Exemple 2 Effet inhibiteur de l'association « GLZ + pentamère (NAG-Glucuronic acid)5 + rétinol » sur la néosynthèse d'acide hyaluronique par des kératinocytes humains normaux.

D'après l'art antérieur, il est admis qu'un équilibre pré-existe entre la néosynthèse et la dégradation de l'acide hyaluronique. En d'autres termes, la néosynthèse de l'acide hyaluronique est le reflet de sa dégradation : mesurer les variations de l'une revient donc à mesurer les variations de l'autre. Pour des raisons de simplicité technique, nous avons choisi de mesurer l'évolution de la néosynthèse d'acide hyaluronique en présence de l'association « GLZ + pentamère (NAG-Glucuronic acid)5 + rétinol », par rapport au contrôle correspondant.

L'isolement des kératinocytes adultes humains (KHN) est réalisé à partir d'un fragment de peau humaine collecté après une opération de plastie abdominale (sujet CAOL, âgé de 38 ans).
Les KHN sont cultivés jusqu'à confluence en monocouche dans des plaques 24 puits et repiqués. Les KHN sont utilisés à la suite du troisième passage.
Le milieu de culture utilisé est le milieu MCK à 37°C dans une atmosphère humide contenant 5% de CO2.
Le milieu de culture MCK est un milieu SFM-defined keratinocytes comportant des facteurs de croissance, auxquels sont additionnées de la pénicilline (50 UI/ml) et de la streptomycine (50 µg/ml).

### Préparation des réactifs :

Le pentamère (NAG-Glucuronic acid)5 est dissout à 2 mg/ml dans le milieu de culture MIK (milieu MCK contenant 2µCi/ml de glucosamine radiomarquée). Il est ensuite dilué en milieu MIK contenant 0,2% de DMSO.

Ce produit est testé à 0,1-0,5 et 2 mg/ml.
- La glycyrrhizine (GLZ) est dissous à 400 mM en DMSO. Elle est ensuite diluée dans le milieu de culture MIK (milieu MCK contenant 2 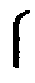 Ci/ml de glucosamine radiomarquée).

Ce produit est testé à 100 - 400 et 800 µM (concentration en DMSO conservée constante : 0,2%).
- Le rétinol est dissous à 10 mM en DMSO. Il est ensuite dilué dans le milieu de culture MIK (milieu MCK contenant 2µCi/ml de glucosamine radiomarquée).

Ce produit est testé à 1 - 10 et 100 nM (concentration en DMSO conservée constante : 0,2%).
- L'association « GLZ + pentamère (NAG-Glucuronic acid)5 + rétinol » est réalisée par mélange des solutions-mères préparées ci-dessus. Elle est ensuite diluée en milieu MIK (milieu MCK contenant 2 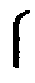 Ci/ml de glucosamine radiomarquée).

Ce produit est testé à :
- 100 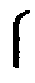 M de GLZ + 0,1 mg/ml de pentamère (NAG-Glucuronic acid)5 + 1 nM de rétinol
- 400 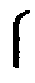 M de GLZ + 0,5 mg/ml de pentamère (NAG-Glucuronic acid)5 + 10 nM de rétinol
- 800 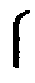 M de GLZ + 2 mg/ml de pentamère (NAG-Glucuronic acid)5 + 100 nM de rétinol

Ces trois solutions ont une concentration constante en DMSO de 0,2%.

### Protocole expérimental : (selon Pienimaki et al, The Journal of Biological Chemistry (2001)Vol276,N°23, June8, p20428-20435)

Le système réactionnel est constitué de kératinocytes humains normaux en culture monocouche en plaques 24 puits (cellules à confluence au démarrage de l'incubation).
Les KHN sont incubés en présence des produits à l'essai et de glucosamine radiomarquée pendant 6-12-24 et 48 heures, sous un volume final de 400 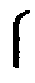 1 de milieu par puits de culture. La température d'incubation est de 37°C, l'atmosphère humide contient 5 % de CO2.

### a. Récupération de tous les GAG comportant de la glucosamine radiomarquée :

A la fin de l'incubation, les milieux de culture sont récupérés par centrifugation; puis on rince les tapis cellulaires avec du PBS. On récupère le mélange composé du milieu de rinçage et du surnageant initial. Après ajout d'acide hyaluronique (10 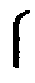 g/fraction, rôle de « carrier » lors des précipitations au Cétyl Pyridinium Chloride (CPC) et de papaïne (200 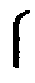 g/fraction, hydrolyse des protéoglycannes avec libération des GAG), les fractions sont incubées 2 heures à 60°C ; puis on incube les fractions 10 minutes à 100°C pour inactiver les enzymes par dénaturation thermique. Enfin les GAG sont précipités par ajout de CPC (concentration finale de 1%) et incubés une nuit à température ambiante. Après centrifugation 10 minutes à 15000g, puis élimination des surnageants contenant la glucosamine radiomarquée non incorporée; on rince les culots par du CPC 1%; et on centrifuge de nouveau 10 minutes à 15000 g puis on élimine les surnageants.

### b. Mesure de la néosynthèse d'acide hyaluronique :

On hydrolyse spécifiquement l'acide hyaluronique contenu dans les culots de centrifugation par incubation (3 heures à 37°C) de ces culots avec de la hyaluronidase (hyaluronan lyase de Streptomyces hyalurolyticus, 0,25 unité /fraction). Pour cela, on incube le milieu contenant ces culots et la hyaluronidase pendant 3 heures à température ambiante, puis on centrifuge 10 minutes à 15000 g. Enfin on précipite les GAG non hydrolysés par ajout de CPC (1% en concentration finale) en présence de chondroitine sulfate (50 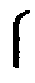 g/fraction- rôle de «carrier»).

La radioactivité du surnageant, déterminée en scintillation liquide (compteur ß), est directement proportionnelle à la quantité d'acide hyaluronique néosynthétisé lors de l'incubation des cellules avec la glucosamine radiomarquée.

Les mesures de radioactivité sont présentées dans les tableaux suivants :

**Tableau 1. : Mesure de la néosynthèse d'acide hyaluronique par des kératinocytes humains normaux en absence de l'association « GLZ + pentamère (NAG-Glucuronic acid)5 + rétinol » (Témoin).**

| | Incubation | Incubation | Incubation | Incubation | incubation |
|---|---|---|---|---|---|
| | 6h | 12h | 24h | 48h | 72h |
| dpm | 4156 | 12216 | 38404 | 62938 | 64701 |
| | 3997 | 11874 | 34050 | 70271 | 74910 |
| | 4232 | 12463 | 30264 | 62411 | 79646 |
| Moyenne | 4128 | 12184 | 34239 | 65207 | 73086 |
| Ecart-type | 120 | 296 | 4073 | 4394 | 7638 |

**Tableau 2. : Mesure de la néosynthèse d'acide hyaluronique par des kératinocytes humains normaux en présence de l'association « GLZ + pentamère (NAG-Glucuronic acid)5 + rétinol ».**

| | | | | | |
|---|---|---|---|---|---|
| 100 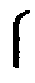 M de GLZ + 0,1 mg/ml de pentamère (NAG-Glucuronic acid)5 + 1 nM de rétinol | | | | | |

| | Incubation | Incubation | Incubation | Incubation | Incubation |
|---|---|---|---|---|---|
| | 6h | 12h | 24h | 48h | 72h |
| dpm | 3508 | 12844 | 28289 | 51974 | 63822 |
| | 3388 | 14053 | 26971 | 55088 | 64251 |
| | 3697 | 13347 | 30200 | 56652 | 61504 |
| Moyenne | 3531 | 13415 | 28487** | 54571** | 63192** |
| Ecart-type | 156 | 607 | 1624 | 2381 | 1478 |
| 400 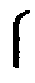 M de GLZ + 0,5 mg/ml de pentamère (NAG-Glucuronic acid)5 + 10 nM de rétinol | | | | | |

| | Incubation | Incubation | Incubation | Incubation | Incubation |
|---|---|---|---|---|---|
| | 6h | 12h | 24h | 48h | 72h |
| dpm | 3672 | 9847 | 25363 | 26813 | 54202 |
| | 3698 | 10724 | 27404 | 36235 | 49799 |
| | 3461 | 10577 | 25351 | 29007 | 54488 |
| Moyenne | 3610 | 10383*** | 26039*** | 30685*** | 52830*** |
| Ecart-type | 130 | 470 | 1182 | 4930 | 2629 |
| 800 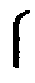 M de GLZ + 2 mg/ml de pentamère (NAG-Glucuronic acid)5 + 100 nM de rétinol | | | | | |

| | Incubation | Incubation | Incubation | Incubation | Incubation |
|---|---|---|---|---|---|
| | 6h | 12h | 24h | 48h | 72h |
| dpm | 3203 | 8263 | 17640 | 32714 | 33910 |
| | 3401 | 8875 | 19682 | 30070 | 29693 |
| | 2580 | 7590 | 21320 | 29092 | 29194 |
| Moyenne | 3061 | 8243*** | 19547*** | 30625*** | 30932*** |
| Ecart-type | 428 | 643 | 1844 | 1874 | 2591 |

| | | | | | |
|---|---|---|---|---|---|
| ** : moyenne significativement différente de celle du groupe Témoin (p<0,05) *** : moyenne significativement différente de celle du groupe Témoin (p<0,01) | | | | | |

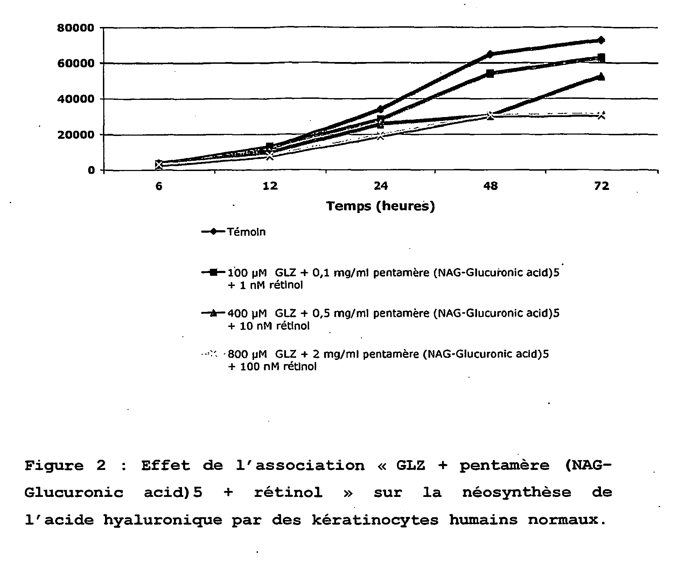

L'effet inhibiteur de l'association « GLZ + pentamère (NAG-Glucuronic acid)5 + rétinol » sur la néosynthèse d'acide hyaluronique par des kératinocytes humains normaux est illustré sur la figure 2.

Les résultats obtenus montrent une inhibition marquée de la néosynthèse d'acide hyaluronique. Cet effet est dose-dépendant et, aux concentrations des trois composants les plus élevées, l'inhibition de la néosynthèse est de l'ordre de 50 à 60%. En raison de l'équilibre pré-existant entre néosynthèse et dégradation de l'acide hyaluronique, cette inhibition de la néosynthèse observée correspond à une inhibition de la dégradation équivalente.

Nous pouvons donc conclure que l'effet de l'association protège l'acide hyaluronique de la dégradation. Ce système permet donc d'augmenter la biostabilité de l'acide hyaluronique et, par conséquent, d'améliorer sa biodisponibilité.

### Exemple 3 Composition n°1

### Solution injectable n°1 contenant les 4 composants

Cette composition est préparée de manière classique pour l'homme du métier:

| | |
|---|---|
| Acide hyaluronique | 2% |
| Glycyrrhizine | 0,02% |
| pentamère (NAC-Glucuronic acid)5 | 0,002% |
| Rétinol | 0,00001% |
| Eau | qsp 100% |

### Exemple 4 : Composition n°2

**Solution injectable n°2 d'acide hyaluronique couplée à une crème contenant les 3 autres composants**

| | *Solution injectable* | |
|---|---|---|
| | Acide hyaluronique | 2% |
| | Eau | qsp 100% |

| | *Crème* | |
|---|---|---|
| | Glycyrrhizine | 0,02% |
| | pentamère (NAC-Glucuronic acid)5 | 0,002% |
| | Rétinol | 0,00001% |
| | Acide stéarique | 3,00% |
| | mélange de monostéarate de glycéryle et de stéarate de PEG (100 OE) | 2,5% |
| | Stéarate de PEG (20 OE) | 1,0% |
| | Cyclopentadiméthylsiloxane | 10,00% |
| | Huiles végétales | 7,00% |
| | Huiles synthétiques | 6,00% |
| | Gomme de silicone | 0,20% |
| | Alcool stéarique | 1,00% |
| | Eau | qsp 100% |

## Revendications

1. Préparation pharmaceutique ou cosmétique comprenant, dans un milieu physiologiquement acceptable, de l'acide hyaluronique, **caractérisée en ce qu'**elle comprend en outre au moins :
- un rétinoïde et/ou ses sels et/ou ses esters, tels que le rétinyl palmitate, le rétinyl acétate, le rétinyl stéarate, le rétinyl oléate, le rétinyl propionate ou le rétinyl linoléate,
- un pentamère de l'acide hyaluronique, et
- un inhibiteur de la dégradation de l'acide hyaluronique qui est choisi parmi la glycyrrhizine ou l'acide glycyrrhétinique, leurs sels, énantiomères et/ou racémates, pris seuls ou en mélange.

2. Préparation selon la revendication 1, **caractérisée en ce que** le rétinoïde est le rétinol.

3. Préparation selon l'une quelconque des revendications 1 à 2, **caractérisée en ce qu'**elle est formulée pour une application par voie topique.

4. Préparation selon l'une des revendications 1 à 2, **caractérisée en ce qu'**elle est formulée pour une appplication par voie parentérale.

5. Préparation parentérale selon la revendication 4, **caractérisée en ce qu'**elle se présente sous forme de solutions ou suspensions pour perfusion ou pour injection.

6. Produit contenant :
- de l'acide hyaluronique,
- au moins un rétinoïde et/ou ses sels et/ou ses esters, tels que le rétinyl palmitate, le rétinyl acétate, le rétinyl stéarate, le rétinyl oléate, le rétinyl propionate ou le rétinyl linoléate,
- au moins un pentamère de l'acide hyaluronique et
- au moins un inhibiteur de la dégradation de l'acide hyaluronique qui est choisi parmi la glycyrrhizine ou l'acide glycyrrhétinique, leurs sels, énantiomères et/ou racémates, pris seuls ou en mélange,
comme produit de combinaison pour une utilisation simultanée, séparée ou étalée dans le temps dans le traitement d'affections dermatologiques.

7. Produit de combinaison selon la revendication 6, **caractérisé en ce qu'**il comprend une composition A comprenant l'acide hyaluronique sous forme de solution injectable, associée à une composition B comprenant au moins :
- un inhibiteur de la dégradation de l'acide hyaluronique qui est choisi parmi la glycyrrhizine ou l'acide glycyrrhétinique, leurs sels, énantiomères et/ou racémates, pris seuls ou en mélange,
- un pentamère de l'acide hyaluronique, et
- un rétinoide et/ou ses sels et/ou ses esters, tels que le rétinyl palmitate, le rétinyl acétate, le rétinyl stéarate, le rétinyl oléate, le rétinyl propionate ou le rétinyl linoléate, sous forme de composition pour application topique.

8. Procédé de fabrication d'une préparation pharmaceutique ou cosmétique selon l'une des revendications 1 à 5, **caractérisé en ce qu'**il comprend l'étape de mélange d'une quantité efficace d'acide hyaluronique, d'au moins un rétinoïde et/ou ses sels et/ou ses esters, tels que le rétinyl palmitate, le rétinyl acétate, le rétinyl stéarate, le rétinyl oléate, le rétinyl propionate ou le rétinyl linoléate, d'au moins un pentamère de l'acide hyaluronique, et d'au moins un inhibiteur de la dégradation de l'acide hyaluronique qui est choisi parmi la glycyrrhizine ou l'acide glycyrrhétinique, leurs sels, énantiomères et/ou racémates, pris seuls ou en mélange.

9. Procédé de fabrication d'un produit selon la revendication 7, **caractérisé en ce qu'**il comprend :
- une première étape de préparation d'une solution injectable, comprenant le mélange de l'acide hyaluronique avec un milieu physiologiquement acceptable,
- une seconde étape de préparation d'une composition adaptée à la voie topique, comprenant le mélange d'au moins un rétinoïde et/ou ses sels et/ou ses esters, tels que le rétinyl palmitate, le rétinyl acétate, le rétinyl stéarate, le rétinyl oléate, le rétinyl propionate ou le rétinyl linoléate avec au moins un pentamère de l'acide hyaluronique et au moins un inhibiteur de la dégradation de l'acide hyaluronique qui est choisi parmi la glycyrrhizine ou l'acide glycyrrhétinique, leurs sels, énantiomères et/ou racémates, pris seuls ou en mélange, dans un milieu physiologiquement acceptable.

10. Utilisation d'une préparation selon l'une des revendications 1 à 5 pour la fabrication d'un médicament destiné au traitement et/ou à la prévention des affections dermatologiques.

11. Utilisation d'une préparation selon l'une des revendications 1 à 5 pour la fabrication d'un médicament destiné à être utilisé en chirurgie réparatrice.

12. Utilisation d'une préparation selon l'une des revendications 1 à 5 pour la fabrication d'un médicament destiné au traitement par comblement des rides, des ridules, des déplétions fibroblastiques et des cicatrices.

## Claims

1. Pharmaceutical or cosmetic preparation comprising, in a physiologically acceptable medium, hyaluronic acid, **characterized in that** it also comprises at least:
- one retinoid and/or salts thereof and/or esters thereof, such as retinyl palmitate, retinyl acetate, retinyl stearate, retinyl oleate, retinyl propionate or retinyl linoleate,
- a hyaluronic acid pentamer, and
- an inhibitor of hyaluronic acid degradation which is chosen from glycyrrhizin or glycyrrhetinic acid, and salts, enantiomers and/or racemates thereof, taken alone or as a mixture.

2. Preparation according to Claim 1, **characterized in that** the retinoid is retinol.

3. Preparation according to either one of Claims 1 and 2, **characterized in that** it is formulated for topical application.

4. Preparation according to either of Claims 1 and 2, **characterized in that** it is formulated for parenteral application.

5. Parenteral preparation according to Claim 4, **characterized in that** it is in the form of solutions or suspensions for perfusion or for injection.

6. Product containing:
- hyaluronic acid,
- at least one retinoid and/or salts thereof and/or esters thereof, such as retinyl palmitate, retinyl acetate, retinyl stearate, retinyl oleate, retinyl propionate or retinyl linoleate,
- at least one hyaluronic acid pentamer, and
- at least one inhibitor of hyaluronic acid degradation which is chosen from glycyrrhizin or glycyrrhetinic acid, and salts, enantiomers and/or racemates thereof, taken alone or as a mixture,
as a combination product for simultaneous, separate or sequential use in the treatment of dermatological conditions.

7. Combination product according to Claim 6, **characterized in that** it comprises a composition A comprising hyaluronic acid in the form of an injectable solution, combined with a composition B comprising at least:
- one inhibitor of hyaluronic acid degradation which is chosen from glycyrrhizin or glycyrrhetinic acid, and salts, enantiomers and/or racemates thereof, taken alone or as a mixture,
- a hyaluronic acid pentamer, and
- a retinoid and/or salts thereof and/or esters thereof, such as retinyl palmitate, retinyl acetate, retinyl stearate, retinyl oleate, retinyl propionate or retinyl linoleate,
in the form of a composition for topical application.

8. Process for the manufacture of a pharmaceutical or cosmetic preparation according to one of Claims 1 to 5, **characterized in that** it comprises the step of mixing an effective amount of hyaluronic acid, at least one retinoid and/or salts thereof and/or esters thereof, such as retinyl palmitate, retinyl acetate, retinyl stearate, retinyl oleate, retinyl propionate or retinyl linoleate, at least one hyaluronic acid pentamer, and at least one inhibitor of hyaluronic acid degradation which is chosen from glycyrrhizin or glycyrrhetinic acid, and salts, enantiomers and/or racemates thereof, taken alone or as a mixture.

9. Process for the manufacture of a product according to Claim 7, **characterized in that** it comprises:
- a first step of preparing an injectable solution, comprising mixing the hyaluronic acid with a physiologically acceptable medium,
- a second step of preparing a composition suitable for topical administration, comprising mixing at least one retinoid and/or salts thereof and/or esters thereof, such as retinyl palmitate, retinyl acetate, retinyl stearate, retinyl oleate, retinyl propionate or retinyl linoleate, with at least one hyaluronic acid pentamer and at least one inhibitor of hyaluronic acid degradation which is chosen from glycyrrhizin or glycyrrhetinic acid, and salts, enantiomers and/or racemates thereof, taken alone or as a mixture, in a physiologically acceptable medium.

10. Use of a preparation according to one of Claims 1 to 5, for the manufacture of a medicament for use in the treatment and/or prevention of dermatological conditions.

11. Use of a preparation according to one of Claims 1 to 5, for the manufacture of a medicament for use in repair surgery.

12. Use of a preparation according to one of Claims 1 to 5, for the manufacture of a medicament for use in the treatment by filling of wrinkles, fine lines, fibroblast depletions and scars.

## Patentansprüche

1. Pharmazeutische oder kosmetische Zubereitung, die Hyaluronsäure in einem physiologisch annehmbaren Medium umfasst, **dadurch gekennzeichnet, dass** sie außerdem mindestens Folgendes umfasst:
- ein Retinoid und/oder dessen Salze und/oder dessen Ester, wie Retinylpalmitat, Retinylacetat, Retinylstearat, Retinyloleat, Retinylpropionat oder Retinyllinoleat,
- ein Pentamer der Hyaluronsäure und
- einen Inhibitor des Abbaus von Hyaluronsäure, der ausgewählt wird aus Glycyrrhizin oder Glycyrrhizinsäure, deren Salzen, Enantiomeren und/oder Racematen, die einzeln oder im Gemisch verwendet werden.

2. Zubereitung nach Anspruch 1, **dadurch gekennzeichnet, dass** es sich bei dem Retinoid um Retinol handelt.

3. Zubereitung nach einem der Ansprüche 1 bis 2, **dadurch gekennzeichnet, dass** sie für die Anwendung auf topischem Weg formuliert ist.

4. Zubereitung nach einem der Ansprüche 1 bis 2, **dadurch gekennzeichnet, dass** sie für die Anwendung auf parenteralem Weg formuliert ist.

5. Parenterale Zubereitung nach Anspruch 4, **dadurch gekennzeichnet, dass** sie in Form von Lösungen oder Suspensionen zur Perfusion oder zur Injektion dargereicht wird.

6. Produkt, enthaltend:
- Hyaluronsäure
- mindestens ein Retinoid und/oder dessen Salze und/oder dessen Ester, wie Retinylpalmitat, Retinylacetat, Retinylstearat, Retinyloleat, Retinylpropionat oder Retinyllinoleat,
- mindestens ein Pentamer der Hyaluronsäure und
- mindestens einen Inhibitor des Abbaus von Hyaluronsäure, der ausgewählt wird aus Glycyrrhizin oder Glycyrrhizinsäure, deren Salzen, Enantiomeren und/oder Racematen, die einzeln oder im Gemisch verwendet werden,
als Kombinationsprodukt zur gleichzeitigen, getrennten oder zeitlich versetzten Verwendung bei der Behandlung dermatologischer Erkrankungen.

7. Kombinationsprodukt nach Anspruch 6, **dadurch gekennzeichnet, dass** es eine Zusammensetzung A, die Hyaluronsäure umfasst, in Form einer injizierbaren Lösung umfasst, zu der sich eine Zusammensetzung B gesellt, die mindestens Folgendes umfasst:
- einen Inhibitor des Abbaus von Hyaluronsäure, der ausgewählt wird aus Glycyrrhizin oder Glycyrrhizinsäure, deren Salzen, Enantiomeren und/oder Racematen, die einzeln oder im Gemisch verwendet werden,
- ein Pentamer der Hyaluronsäure und
- ein Retinoid und/oder dessen Salze und/oder dessen Ester, wie Retinylpalmitat, Retinylacetat, Retinylstearat, Retinyloleat, Retinylpropionat oder Retinyllinoleat,
in Form einer Zusammensetzung für die topische Verabreichung.

8. Verfahren zur Herstellung einer pharmazeutischen oder kosmetischen Zubereitung nach einem der Ansprüche 1 bis 5, **dadurch gekennzeichnet, dass** man in einem Schritt Folgendes mischt: eine wirksame Menge von Hyaluronsäure, mindestens einem Retinoid und/oder dessen Salzen und/oder dessen Estern, wie Retinylpalmitat, Retinylacetat, Retinylstearat, Retinyloleat, Retinylpropionat oder Retinyllinoleat, mindestens ein Pentamer der Hyaluronsäure und mindestens einen Inhibitor des Abbaus von Hyaluronsäure, der ausgewählt wird aus Glycyrrhizin oder Glycyrrhizinsäure, deren Salzen, Enantiomeren und/oder Racematen, die einzeln oder im Gemisch verwendet werden.

9. Verfahren zur Herstellung eines Produkts nach Anspruch 7, **dadurch gekennzeichnet, dass** man
- in einem ersten Schritt eine injizierbare Lösung herstellt, die das Gemisch von Hyaluronsäure mit einem physiologisch annehmbaren Medium umfasst,
- in einem zweiten Schritt eine an den topischen Verabreichungsweg angepasste Zusammensetzung herstellt, die das Gemisch von mindestens einem Retinoid und/oder dessen Salzen und/oder dessen Estern, wie Retinylpalmitat, Retinylacetat, Retinylstearat, Retinyloleat, Retinylpropionat oder Retinyllinoleat, mit mindestens einem Pentamer der Hyaluronsäure und mindestens einem Inhibitor des Abbaus der Hyaluronsäure, der ausgewählt wird aus Glycyrrhizin oder Glycyrrhizinsäure, deren Salzen, Enantiomeren und/oder Racematen, die einzeln oder im Gemisch verwendet werden, in einem physiologisch annehmbaren Medium umfasst.

10. Verwendung einer Zubereitung nach einem der Ansprüche 1 bis 5 zur Herstellung eines Medikaments für die Behandlung und/oder Vorbeugung dermatologischer Erkrankungen.

11. Verwendung einer Zubereitung nach einem der Ansprüche 1 bis 5 zur Herstellung eines Medikaments für die Verwendung bei der plastischen Chirurgie.

12. Verwendung einer Zubereitung nach einem der Ansprüche 1 bis 5 zur Herstellung eines Medikaments für die Behandlung durch Auffüllen von Falten, Fältchen, Fibroblastenverarmungen und Narben.
